# EUROPEAN PATENT APPLICATION

(11) **EP 1 961 433 A1**
(43) Date of publication of application: **27.08.2008**
(21) Application number: 07102748.6
(22) Date of filing: 20.02.2007
(51) Int. Cl.: A61L 27/56, A61F 2/44

(54) **Porous substrates for implantation**

(71) Applicant: National University of Ireland Galway, Galway (IE)
(72) Inventor: Apatsidis, Dimitrios Dr., London, IG4 5EE (GB); Ryan, Garrett, Galway (IE); Pandit, Abhay, Prof., Galway (IE)
(74) Representative: Lane, Cathal Michael

(57) **Abstract**

A porous substrate or implant for implantation into a human or animal body constructed from a structural material and having one or more regions which when implanted are subjected to a relatively lower mechanical loading. The region(s) are constructed with lesser mechanical strength by having a lesser amount of structural material in said region(s) relative to other regions. This is achieved by controlling pore volume fraction in the regions.

## Description

### Field of the Invention

The present invention relates to porous matrices and to porous substrates. In particular, the present invention relates to porous matrices which are suitable for use as implants, such as implants to be connected to bone for example spinal implants and dental implants. Of particular interest are porous matrices having controlled morphology. Typically the porous matrices of interest are those constructed of biocompatible materials including metallic materials, ceramic materials and polymer materials and combinations thereof. Examples of polymeric materials include polylacetate and polyvinyl alcohol (PVA).

End uses for the porous matrices of the present invention include all applications where mechanical stability is to be imparted to a part of the body, for example where replacement or re-enforcement is required. It is important that such implants are biocompatible in the sense that they do not cause an immediate autoimmune reaction so that the body in which they are implanted does not reject them. Furthermore, it is desirable that the implant is integratable into the body, for example by osseointegration.

### Background to the Invention

Implants, including metallic, ceramic and polymer implants have generally been used to impart mechanical strength to a part of the body by being applied to a weakened part of the body (such as a fracture) and additionally or alternatively being used to replace or repair a part of the body such as a full or partial bone structure.

For example it is known to provide implants which are constructed of titanium, stainless steel, chromium other metals (and including alloys of such metals), ceramics including oxides, non-oxides such as carbides, borides, nitrides and silicides and composite materials. Of particular interest are implants based on hydroxyapatite materials such as ceramic hydroxyapatite materials. Of most interest are implants that fit to bone. This includes synthetic bone, and dental implants. It also includes plates, pins etc utilised to hold relative positions of two parts of the body.

It has been found, for example that with conventional implants stress shielding can occur. Stress shielding is generally considered to be a redistribution of load and consequently stress on a bone) that can occur when an implant replaces a bone, including a portion of a bone such as in a replacement hip or knee. Generally the implant will be stiffer (more stress resistant) than the bone causing stresses typically absorbed by bone to be transmitted by the implant, which does not so readily absorb such stresses. Thus routine movement of the body over time may cause the bone connected to the implant, (whether directly or indirectly such as through a joint) to experience lower stresses than if the implant was not present. This may result in bone degeneration and, consequently, implant loosening. Stress shielding has been found to occur in a significant number of cases, for example in a large percentage of the joints of hip replacement patients after a number of years.

On the other hand, if the material of the implant is not stiff enough to take repeated loadings, early material failure will occur. That is, the implant will absorb more stresses than the part of the body it replaces or to which it is attached. Over time this additional stress absorption may lead to material failure.

US Patent Application No. 2002/0120336 (also published as US Patent No. 6,673,075) to Santilli, describes an intervertebral spacer, which is suitable for use as a spinal implant. It is created of a rigid, porous material. The spacer is strong enough to accommodate loads imposed by adjacent vertebrae and is created with a porous matrix which is intended to facilitate tissue ingrowth and bony fusion. Porosity in the spacer comprises a plurality of randomly sized, substantially interconnected voids, which are disposed throughout the spacer. A number of techniques are described to create the implant. A first technique involves creating a porous metallic fibre mesh, which is formed by interengaging and intertwining strands of the material, which are then sintered together with beads to form the desired rigid shape with a porous matrix. The second method described utilises a blowing agent. The blowing agent is mixed with powdered metal and the mixture is heated to a foaming temperature. Subsequent cooling allows the voids created by the blowing agent to be retained in the metal. A third method described is to form a spacer. A uniform mixture is formed from beads, powdered metal and a binder. The beads are made from wax or other suitable low melting temperature material. The mixture is heated in the mould at a low-temperature to set the binder and burn off the beads. Thereafter the mould is heated to a high sintering temperature to oxidise the binder and solidify the powder. A further method described is to take a solid block of a biologically inert, strong material such as PEEK polymer, titanium, or ceramic and to form a series of openings or pores in the block. The openings or pores are variably sized and variably spaced.

The clear intention of the teaching within this patent application is to create a porous metallic structure with randomised pore shape, pore size, and pore distribution. While more regular pore shape, porous size and pore distribution will be achieved with the third method described in this patent it is still randomised to the extent the relative positioning of the beads is random.

US Patent No. 4,636,219 (Pratt et al) describes a biocompatible mesh grain structure which has a substantially uniform porous size so as to promote substantially uniform bone penetration into the mesh. US Patent No. 5,443, 510 describes a thin layer of metal mesh on the surface of an implant for bonding with a porous surface layer. US Patent No 4,969,904 (Koch et al*)* describes a wire mesh that is welded to a metal substrate with step-like protuberances. The protuberances allow for spot-welding. The mesh facilitates bone cement or bone tissue ingrowth. US Patent No. 5,507,815 discloses a chemical etching method involving masking which provides a random irregular pattern that is adapted to receive the ingrowth of bone material and to provide a strong anchor for that material.

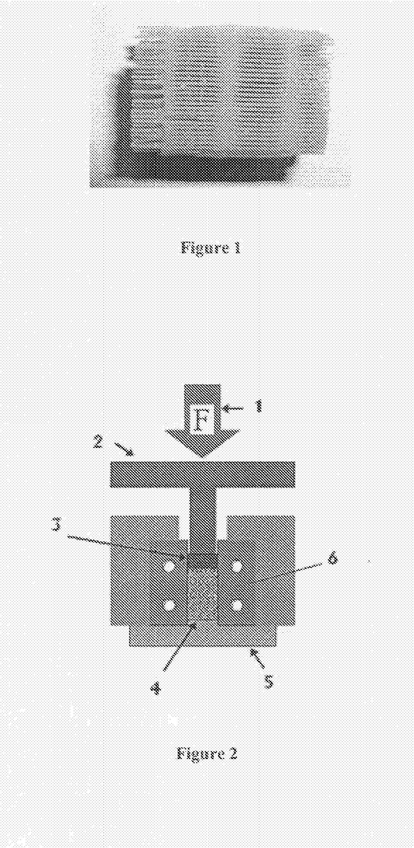

Melican et al "Three-dimensional printing and porous metallic surfaces; a new orthopaedic application" J. Biomed Mater Res 2001; 55;194-202. The authors of this paper described fabricating three experimental textures. Each texture is described as having a surface layer and a deep layer with distinct individual porosities. Three-dimensional printing was utilised for solid free-form fabrication techniques to generate ceramic moulds by printing binder onto a bed of ceramic powder with the printhead in a rastering motion. The Melican *et al* moulds contained sub-millimetre cavities into which moulds Co-Cr was infiltrated to form non-porous implants with regular porous surfaces that ranged in porosity from 38 % to 67 % (the porosity can only be imparted to the surface by the external mould). These moulds suffer from limitations of accuracy in resolution, particularly due to use of rastering techniques that result in cubiform pores (see for example Figure 2). According to present thinking bone growth is optimised only when pore shapes are approximately spherical and additionally when pore sizes lie in the range of 100-400 µm (see for example Cameron HU, Pilliar RM, and Macnab Biomed Mater Res 1976;10:295-302) I. The implants utilised were threaded implants which were implanted as cylindrical transcortical implants. The ceramic mould is printed directly using the printing techniques described. After printing the ceramic mould is utilised as a cast for the metallic material used. It is generally accepted that the pores formed by this technique must be cubic in form. In other words the dimension of the pore must be the same in all three directions (xyz planes).

Wen et al "Processing of biocompatible porous Ti and Mg" Script. Mat. 2001;45;1147-1153, describe employing metallic foams of Ti and Mg to design a morphology with porosity and pore size. An agate mortar was used to bind spacer particles and the metallic powder. This resulting mix was then compacted to form a green pellet. Organic powders were then removed thermally. The size, shape and quantity of the spaced holder used was employed to control the mechanical properties. Nonetheless, it is not possible to fabricate substrates having scaffold structures with completely open-cell structures therein using this method. In particular the final location of the space holding particles within the mix cannot be adequately controlled. Furthermore contacts between the spaced holding particles is not certain which means the resulting pores may not be connected.

Bram *et al* describe similar methods to Wen *et al* (above) in particular utilising space holder. Bram *et al* describe utilising carbamide (urea) particles of spherical or angular shape and ammonium hydrogen carbonate particles of angular shape together as space-holder materials.

Li et al "Porous Ti6A14V scaffold directly fabricated by 3D fibre deposition technique: Effect of nozzle diameter" J Mater Sci Mater Med 2005;16; 1159-63. Li *et al* described the different direct metal 3D printing technology. A slurry of the Ti6A14V powder was mixed with an aqueous solution of binders and forced through a syringe nozzle by applying air pressure. The slurry was plotted on a flat surface and layered, each layer at 90° to the last. The resulting scaffold was dried for 24 hours at room temperature and sintered under high vacuum at 1200 °C for two hours. This method results in uniform pore size and densities within a matrix. There is no possibility in this technique to vary the shape of the pores.

Tucinskiy et al "Titanium foams for medical applications" in ASM conference on Materials and Processes for Medical Devices; 2003 Anaheim CA; 2003 describe rods constructed of a shell of titanium powder and polymer binder and a core of channel-forming filler which are extruded together. The rods are cut into predetermined lengths and the organic filler of the core was later thermally removed. Cylindrical pores were thus generated in the green pellet resulting from the compacted mix.

Li et al "A novel porous Ti6A14V: Characterization and cell attachment" JBiomed Mater Res 73A; 2005; 223-233, use a polymeric (polyurethane) sponge impregnated with a Ti6A14V slurry prepared from Ti6A14V powders and binders. After sintering at high temperature and high vacuum porous Ti6A14V was produced by removal of the sponge.

Li et al: "Preparation and characterization of porous Ti"; Key Engineering Materials 2002:218:51-54, also describes creating porous structures by immersing polyurethane foams in a Ti slurry mix. This process was repeated until all of the foam was coated with titanium powder. After thermal removal of the polyurethane forms and binder, sintering of the powders was carried out and a resultant reticulated open-cell foam with hollow titanium struts remained.

Lenka Jonasova et al "Hydroxyapatite formation of alkali-treated titanium with different content of Na+ in the surface layer"; Biomaterials 23; 2002; 3095-3101**;** describes creating a bone-like apatite layer on the surface of an implant, such as metallic Ti implants, when treated with NaOH (sodium hydroxide). Such methods are useful in the present invention and the contents of this publication are incorporated herein, in its entirety, by reference.

Notwithstanding the disclosures of the documents acknowledged above, there is still a need to strike a balance between providing implants which cause stress shielding and those which will suffer early material failure. It is also desirable to provide a good platform for bone cell migration. This has the advantage that it allows for good integration of the implant with neighbouring host bodily tissue such as bone. Furthermore, the techniques of the present invention allow the varying of shape of the pores that is advantageous, particularly for adapting to external loads that are not normally uni-axial, but may be experienced from different directions at the same time.

### Summary of the Invention

The present invention provides a novel substrate for use in an implant. Of particular interest are load-bearing implants. The present invention provides products which may be employed in an implant with consequent reduction in the stress-shielding problems described above. The products of the invention are also adapted to facilitate bone-ingrowth.

In general terms the present invention relates to a porous substrate (which may be a whole implant or part of an implant) for implantation into a human or animal body. The substrate is constructed from a structural material and has one or more regions which, in the implanted configuration, is subjected to a relatively lower loading, said region(s) being constructed with lesser mechanical strength. The present inters have realised a method of eliminating unnecessary material from an implant. In particular the present inventors can construct a porous substrate where the mechanical strength imparted to a substrate can be closely controlled. This is done by creating a three-dimensional physical structure (a spacer) (which works as an inverse or negative mould in the sense that the material of which it is made is modelled to the shape of the pore network) to impart the pore structure to the structural material of the substrate. The pore network will have non surface-confined porosity. The desired region(s) may be constructed with lesser mechanical strength comprise a lesser amount of structural material in said region(s) relative to other regions.

In particular the present invention provides a porous substrate for use in a load bearing implant, the substrate comprising:
(i) a load bearing porous structure formed of a load bearing material; and
(ii) pores in the structure,
the substrate further comprising:
(iii) a first region of higher load capacity; and
(iv) a second region of lower load capacity;
the first region being formed with a relatively lower pore volume fraction and the second region being formed with a relatively higher pore volume fraction. In this way the strength of the substrate can be varied by using the pore volume fraction as the controlling parameter. Greater pore volume fraction will mean less load bearing material and *vice versa.* Consequently greater pore volume fraction results in relatively lower strength and *vice versa.* It will be appreciated that regions having a higher fraction of voids will have a lesser structural strength and thus a lower load bearing capacity.
It is desirable that there is a 5% or greater difference in pore volume fraction between given regions, more desirably a 7.5% or greater difference, for example a 10% or greater difference. Indeed within the present invention much greater differences in pore volume fraction may be achieved. For bone integration in particular it has been found to be desirable to have pore sizes in the range of from 100µm to 400µm. It will be appreciated then that pore sizes may be selected from within this range for separate regions allowing a ratio of up to 4:1 in pore size selection. This in turn would mean a 4:1 ratio in pore volume fraction assuming the same number of pores are present per unit volume. Obviously the pore volume fraction can be further increased or decreased by increasing/decreasing the number of pores as desired. A pore size which is convenient for use is about 200 µm as this will allow for ease of overlapping of pores. Open pore networks generally have 3D overlap, that is overlap of pores in the x,y, and z planes. The present invention provides such open pore networks in substrates suitable for load-bearing purposes within the body and in which the position of the pores is controlled.

In the present invention then the load-bearing scaffold can be configured for absorbing more stress loading in the region of lower load capacity thus reducing the transmittance of loading to other parts of the body. It will be appreciated by those skilled in the art that the porous substrate can have the property of having a lower modulus of elasticity (elastic modulus) than an equivalent body part, for example equivalent bone. More particularly regions of the substrate which are generally subjected to lower loading than other regions can have an elastic modulus lower than that of an equivalent body part. Regions to be subjected to higher loadings will generally have an elastic modulus substantially equal to or greater than that of the equivalent body part. This in turn means that while overall the substrate will have sufficient mechanical strength for its task the volume of foreign (implanted) material is substantially reduced. This means that for example the amount of metal used to make an implant can be substantially reduced.

Ideally, where the substrate is replacing a body part the substrate will stay in place to provide the mechanical strength while promoting regeneration of the body part until eventually the substrate is replaced by a regenerated body part. In such a construction the substrate is made from a resorbable material.

The present invention provides a simple yet highly effective structure as it allows the load bearing capacity of different regions of the implant to be varied according to the mechanical load-bearing properties required in a given part of the body. In other words, the mechanical strength required (in any given part of the body) can be modelled or profiled (for loading in different regions of the body part) and the substrate can be manufactured to the desired profile. In simple terms, this means that the substrate can be stronger in regions where more loading is imparted and with less strength in regions where lower relative loadings are imparted. Generally the substrates of the present invention are best employed as compressive load force bearing substrates. It will be appreciated that the implants of the invention however may also be employed to take extension (and shear) loading forces.

Generally the substrates will be formed from a single material (as distinct from employing different materials in different regions) with a substantially uniform density so that the structural strength of the substrate in any given region will be determined by the amount of material present to take the load and is thus controllable utilising pore volume fraction as the controlling parameter.

A further very beneficial advantage of the present invention is that the amount of material required in the substrate can be reduced. Instead of having to make the substrate so as to take relatively high loading tolerances in all regions thereof, the substrate can be adapted to have a lower loading tolerance in certain regions. This in turn means that the amount of structural material (load bearing material) can be reduced in the areas requiring lower loading tolerances. Reduction of the amount of material required is desirable because it reduces cost, reduces the overall weight of the implant, and furthermore reduces the amount (mass) of material implanted in the body with the consequent reduction in the probability of rejection by the immune system of the host body, for example by surrounding an implanted device with a collagen-based material.

Generally speaking, for best integration with bone structures of the body, it is desirable that the substrate has an at least partially open-cell pore structure. More desirably it is the substrate has a fully open-cell pore network. The pore network will desirably extend in the substrate to at least a point of attachment for the substrate to the body part (usually running to at least one surface of the substrate for example a surface which will be arranged in use to be proximate the desired body part). In other words, the pore network will extend from an attachment point on (a surface of) the substrate through the body of the substrate. Closed-cell pore (non-interconnected pore) structures are generally suitable where bio-integration is not required.

Bio-compatible materials such as mesenchymal cells, osteoprogenitor cells which will subsequently differentiate into bone producing osteoblast cells, may be incorporated into the substrates of the present invention. Other materials such as growth factors and bio-glues may be incorporated or added. Growth factors will induce mesenchymal cells and osteoprogenitor cell differentiation into osteoblasts and the like. Material such as collagen or fibrin can be used to provide a sticky surface to which cells may adhere. For example an injectable protein, in any suitable form such as in gel form can be used. For example an osteoconductive carrier such as fibrin may be employed. Fibrin may be generated from fibrinogen and thrombin. Viral vectors may be incorporated into the substrates and may act to deliver genetic material which may encode for biological material such as growth factors or antibodies that will bind to specific cell proteins, thus attracting cells to the implant 30. Recombinant forms of suitable materials may be employed. For example bone Morphogenetic Protein 2 (rhBMP-2) can be employed. Materials can be added to fibrinogen and thrombin so as to form fibrin to incorporate those materials. As will be appreciated materials employed such as fibrin may also contribute to haemostasis following implantation.

Coatings may also be applied for example an apatite layer may be applied. It will be appreciated that all materials may be applied to the entire substrate or to regions thereof. Indeed different materials may be applied to different regions as desired. Apatite layers are expected to enhance biocompatibility and osseointegration following implantation.

An apatite layer may be generated for example by treating the metal in an alkaline material for example sodium hydroxide. This is to create a hydrated oxide (gel) layer on the metal. The substrate may then the heat-treated (for example at 500-700 °C, more particularly about 600 °C) to form an amorphous alkali/metal layer. This layer can then be exposed to SBF (Simulated Body Fluid) resulting in a hydrogel layer including apatite nucleation on the surface.

The present invention can also be considered to relate to a porous substrate for use in a load bearing implant 30, the substrate comprising:
(i) a load bearing scaffold structure formed of a load bearing material; and
(ii) an open-cell pore network defined by pores in the scaffold structure, the substrate further comprising:
(iii) a first region of higher load capacity (mechanical strength); and
(iv) a second region of lower load capacity(mechanical strength);
the first region being formed by a load bearing scaffold structure of relatively greater structural strength and the second region being formed by a load bearing scaffold structure of relatively lower structural strength.

Generally speaking the inventive structures of the present invention may be achieved by using pore shapes of any desired shape. However, it is desirable to use pores of substantially ellipsoid shape. The term ellipsoid is inclusive of spheroid and spherical shapes are of interest within the present invention. Ellipsoid includes both prolate and oblate ellipsoids (generated by rotation of an ellipse about major and minor axes respectively).

Generally it is desirable that the major axis of the ellipsoid(s) forming the pores be arranged substantially transverse to a load bearing axis of the substrate. In other words the (axis of the) flatter orientation of the ellipsoid is arranged substantially perpendicular to a load-bearing direction.

Generally speaking it is desirable that the pore volume fraction in any given region is at least 20%, more desirably 25%, for example 30%.

For different load-bearing regions within the substrate it is desirable that there is at least a 5%, more particularly a 10%, for example a 20% difference in pore volume fraction as between the regions. Where more than two load-bearing regions of different load capacity are employed then desirably the pore volume fraction as between the regions is selected as set out below, the area of reduced strength may have up to 90% pore fraction though in general the pore volume fraction will be in the range from 70 to 90% for example 75 to 85% such as about 80%. For areas of higher loading it is desirable to have lower pore volume for example from about 30 to 65%, such as about 35 to 60%, suitably about 50%.

Within each of the load bearing regions it is desirable that individual pore volume is between 100 and 300 µm more particularly 150 and 350 µm, for example 200 and 400 µm.

With the present invention it is possible to provide channels between pores to connect the pores as distinct from overlapping the pores. The present inventors have found however that to have communicating channels going from each pore to its neighbouring one(s) may result in too stiff a structure, which would not be well suited to preventing stress shielding

A specific design aspect of the present invention is to have pores overlap (each with the next). Desirably an interconnecting opening of 50- 100µm is achieved. It will be appreciated that adjacent pores can thus form a contiguous pore volume. This is important for migration of osteoblasts through the entire substrate. In particular it will be appreciated that the contiguous pore volume can be in Cartesian co-ordinate system x,y or z planes. Generally the z axis is taken to be the direction of loading and it is thus desirable that the contiguous pores connect in the z axis (loading) direction. The pore volume fraction in any given region will desirably remain with the selection limits given above.

The present invention allows fabrication of precisely engineered and modelled open-cell porous materials. Three-dimensional printing can be employed as will be described below. The invention is also applicable in the field of powder metallurgical techniques. The present invention thus provides open-cell porous matrices. These are highly versatile and useful. For example they may be employed as tissue replacement scaffold. Furthermore rapid prototyping can be achieved with the present invention. Products which may be created with the present invention included bone-mimicking scaffolds. Of particular importance with the present invention is the ability to attain functionally-graded pore distribution.

It will be appreciated that the pore structures achievable within the present invention can extend through the entire substrate volume and are not confined to surface regions (such as is achievable where (surface) porosity is imparted only by an external mould). The pore structures of the present invention generally extend substantially through the entire body of the substrate. In particular it is desirable that the porosity extend into the substrate (through the volume thereof) in the load bearing direction thereof (generally the z-axis).

The present invention provides a simple yet highly effective method of preparing porous substrates. The method of the present invention includes: identifying one or more regions within a substrate (to be implanted within the human or animal body) formed of a biocompatible material which will be subjected to lesser loading and reducing the amount of structural material in said regions. The amount of structural material can be reduced by increasing the pore volume fraction in the desired region(s).

One method within the present invention for forming a porous substrate includes the steps of:
(i) selecting within a substrate at least two regions which will have differing load capacity; and
(ii) selecting a pore structure that will impart to the substrate said at least two regions of differing load capacity; and
(iii) applying the pore structure to the substrate.

Such a method is relatively straightforward to implement but allows a huge variety of substrates to be manufactured to any desired requirements. In particular, it is possible to match structural strengths within the substrate to those required within a physiological environment.

More particularly, a physiological model of loading can be employed to determine the physiological loading forces and at least certain of those loading force requirements can be imparted to the substrate using the pores as the controlling parameter. Generally there will be at least two regions within the substrate, one having the ability to take a higher structural loading than the other. The differential in ability to take loading can be imparted by utilising the pores as the controlling (load-bearing strength) parameter. Generally the structural material will have a relatively higher flexibility in areas of greater pore volume fraction (due to reduced thickness of material (around each pore)) though generally in those areas it will have a lesser load capacity. The relative pore positions are controlled and not randomised as in the case of the prior art documents set out above. The pores are set down according to a pre-set 3D pattern.

It will be appreciated that by varying the load-bearing capacity of the substrate, particularly using physiological model link, can allow a person skilled in the art to match the characteristics of the substrate to the requirements of the body this can allow for reduction in shear stresses e.g. stress shielding etc.

It will be appreciated that a requirement can arise (for example within a physiological model) for more than two regions within the substrate to have differing loading capacity (structural strength). The method and substrates of the present invention can match the requirements of loading of the physiological environment to the extent required. It is possible that there is continuous change in structural strength requirement across the substrate and the present invention can match that requirement.

While the substrates of the present invention can be matched to a physiological model it will be appreciated that the substrates of the present invention can be made with any desired porosity (for example simply to reduce the amount of material in the substrate).

Generally a load bearing capacity differential of any desired amount within the structural integrity of the substrate can be accomplished. In general differences of between 10% and 30% will be used widely.
The method of the present invention can include the steps of:
employing a model of the physiological load bearing requirements; and
making the substrate to (at least certain of) the physiological load-bearing requirements.

Again this is a relatively straightforward process that can be adapted with ease to make substrates for different physiological requirements.

In modelling the physiological load bearing requirements (for a site) within the body the regions which experience relatively greater physiological loading forces than others and those regions can be identified. The load bearing requirements can thus be mapped into the substrate as desired.

The present inventors have made yet further developments to a process within the present invention.

In particular the inventors have noted that it is possible to implement the present invention by providing a spacer which is in the form of the pore structure to be applied to the substrate. This spacer can be made to embody all requirements of the pore structure. In particular the spacer will be a unitary 3D array of spacer material which has a predefined array of spacer material which takes up the pore structure, and the space of the array unfilled by spacer material represents space to be taken up by the material of the substrate.

Prior art techniques including the use of spacer materials, for example Bram *et al* above, rely on the random positioning of particles to achieve a porosity. While the overall pore volume fraction can be controlled, to an extent, utilising the number of particles as the controlling parameter, the distribution of the particles is random so that the final pore structure achieved, and the final pore volume fraction achieved is not fully controlled.

The predefined spacer will incorporate individual, (and thus overall,) pore shape, size and position. This means that the relative position of the pores is fixed.

The spacer can be considered to be a negative of the substrate in the sense that the spacer material takes up the space which represents the pore structure for the substrate (that is the filled space represents voids in the (final fabricated) substrate). The unfilled space of the spacer represents the space to be taken up by structural material of the substrate to form the substrate.

While Wen *et al* (above) describe foams, and Li *et al* describe polymeric sponges, each to achieve porosity, it is clear that neither of these methods allow control of pore size or distribution, as with foams the final structure depends on the degree of foaming while in the sponge technique the porosity is determined by the PVC sponge initially employed. In the latter case the pore size is loosely based on the pores within the sponge (all pores will reduce in size because of the coating process and some pores will partially or completely fill) and taking into account the thickness of coating etc. It is clear Wen and Li are looking for random porosity to mimic naturally occurring porous structures.

Once the spacer has been constructed, structural material (or components to form the structural material) to form the substrate can be infiltrated about the spacer, (and if required (the spacer material composite) placed within a mould).

The spacer (3D array or scaffold) can be constructed of any suitable material. Desirably the material is one that can be set down by three-dimensional modelling systems such as three-dimensional printing techniques. This means that for example computer models are easily used to produce the spacer. It is a convenient method of implementing the method of the present invention therefore, to take a model of the required porous structure, and to create a spacer representing the porous structure using printing techniques. It will be appreciated then that the spacer can be created to provide a (resultant) substrate structure which will have the desired porosity (and thus loading) profile. An alternative method is to use cutting techniques, or selective sintering for example selective laser sintering (using lasers to selectively sinter target areas), to create a three-dimensional scaffold.

One method of creating the porous structure is to place the structural material (or components to form the structural material) about the spacer. This requires having the structural material or its precursor in a form which can take up the space around the spacer. For example the structural material may take a particulate form for example a powder form (for example for metals including metal alloys, ceramics, and polymeric material). Alternatively, the structural material may take a liquid form, which includes suspensions such as slurries. Generally, a surrounding mould will be employed about the spacer to retain the structural material or its precursor to a desired (exterior) shape.

Slurries of insoluble particles of the structural material can be employed if desired. Aqueous slurries may be employed. Slurries in other solvents may also be employed. For example one or more of the following solvents may be employed; ethylene glycol, di ethylene glycols and combinations thereof.

Suitable metals including stainless steel and titanium, and titanium alloys such as Ti-6A1-4V. Where titanium is employed it is desirable to infiltrate the spacer with titanium in slurry form. One suitable slurry is that of titanium particles (powder) carried in ethylene glycol. Drying may be required before compaction can take place where slurries are used. The materials can be left to dry or drying can be accelerated using heating and/or air blowing etc.

Compaction, or some other such method, may be employed to confer desired load-bearing properties on the structural material or its precursor and to thus form the substrate. Additional steps will be completed where required to confer the desired load-bearing properties. For example where metal or alloys are used additional steps such as sintering may be required to harden the metal in the desired shape. If desired, sintering may be done under a vacuum.

The spacer may be made of any suitable material, for example plastics material including nylons for example nylon 6,6, wax material including: paraffin wax (those with low ash content (~0.1 %)). The wax materials include those optimised for printing, for example those based on paraffin waxes. Suitable (printable) wax materials include those supplied by 3D Systems Inc., for example those as described in US Patent No.s 6,989,225 and 4,575,330 and 5,234,636 the contents of each of which are expressly incorporated herein by reference.

In this context a wax material will generally be easily malleable at room temperature, have a melting point of greater than 45°C, and a low viscosity when melted and be thermoplastic. Such properties are useful selection criteria for materials for forming a spacer of the present invention. Thermoset materials may also be employed. Thermosets (generally used in powder form) will melt when heated, but only up to a certain point. Thereafter, they will cross-link and harden and, further heating will not re-melt them. However the present inventors have found that thermosets are materials that can be easily removed, for example by employing organic solvents, such as Xylene (which may increase the volatility of the thermoset). This allows the inventors to completely remove any traces of this material that is used as a spacer for the porous matrix fabrication process. In this way it can be ensured that it will not have any interactions with the biological host environment.

Suitable materials for forming the spacer include those described above. Exemplary materials from within these types include those thermoset materials commercially available materials include Thermojet® waxes available from 3D Systems Inc.

Irrespective of how the spacer is formed, it may be necessary to remove the spacer from within the substrate, once the substrate has been formed with a pore network of that of the spacer to open up the pores.

It is desirable that the material of the spacer is a thermoplastic material. While plastic materials such as nylon may be employed, generally plastic polymeric materials have a melting point in excess of 100° C. The present inventors have found that higher melting point materials (melting point in excess of 100° C) may be difficult to remove without leaving residues. Accordingly spacers made from such materials are less desirable. The present inventors have found that more suitable materials are those having a melting point less than about 100° C. In any event any spacer material that can be washed out without leaving any significant residue is suitable for use within the present invention. Easy to dissolve materials (in solvent), optionally when heated, are of interest.

A further problem solved by the present invention is an issue which may occur during the formation of the substrate. In particular, during compaction processes, the spacer itself may become somewhat compressed (any such compression will of course be factored into the final pore size requirement). Particulate matter, which is compacted about the spacer, can undergo shear forces which result from the spacer resizing when the compaction force is removed. This means that the entire construct (spacer plus control material) can fail due to the formation of cracks or the displacement of the structural material. This problem arises for example where metal powders are used to form the structural material. Such metal powders will generally only be held together by physical interactions resulting from the compaction forces. The "spring back" of the spacer (resulting from its viscoelastic material properties) can thus quite easily disrupt the integrity of the construct, for example by causing cracks to appear. Such a construct (spacer and structural material) is often referred to as a "green compact". Subsequent heat treatment (sintering) is utilised to cause the metal particles to adhere to each other, thus forming the substrate with the desired mechanical strength.

The present inventors have found that by heating the construct while it is being (repeatedly) compacted or while held under (a constant) compaction force, the desired substrate can be formed while the spacer material can melt or dissolve away. This was found to eliminate any built up stresses and remove the possibility of the problem of loss of integrity arising. Generally the construct will be heated only to a temperature below the melting point of the spacer material. Generally the heat applied will be sufficient to make the spacer material more pliable. For example the surface of the spacer material may be caused to melt.

The inventors further found that by using displacement control which set a constant compaction force (as distinct from a force control) on the compaction press used for compaction more reliable compaction without loss of integrity was realised.

Any residual spacer material will desirably be removed by washing (in addition to or as an alternative to heating) for example by using an appropriate solvent, such as an organic solvent. Suitable solvents include the following: xylene, and other benzene derived solvents such as toluene and combinations thereof. The washing may be repeated a number of times to ensure sufficient removal of the spacer material.
Washing can be carried out by immersion of the construct in a solvent bath or the like.

The spacer itself forms part of the present invention. In general terms the spacer of the invention will be for forming a porous substrate for implantation into a human or animal body the spacer being a three-dimensional array of spacer material for imparting a pore structure to structural material forming the substrate. The three-dimensional array of spacer material is configured to impart a higher pore volume fraction to a first region of the substrate and to impart a region of lower pore volume fraction to a second region of the substrate.

It is clear that the spacer is a pre-formed representation of a desired pore structure. The pore structure is fixed when the spaced is made. This contrasts with prior art where the pores are created in situ by the random dispersion of particles within the material forming the substrate. Generally the spacer of the invention will not comprise any material of which the substrate is to be formed.

The spacer will be internal to the substrate in the sense that material forming the substrate will be infiltrated in and through the spacer so that substantially all of the space within the boundary of the spacer will be filled with material to form the substrate. Indeed the free space in the spacer will represent the scaffold structure that can then be formed utilising the spacer.

The pores in the spacer can be formed by setting down struts which define the pores by arranging the struts in an alternating diverging and converging arrangement for example in a repeating chevron pattern. In this way substantially ellipsoid shapes can be formed. It is to be noted that compaction forces and indeed heating effects on the spacer will tend to contribute to pores (within the final substrate) which are closer to being of the exact shape desired.

The invention extends to a porous substrate obtainable by the methods of the present invention.

Either during or after its manufacture, the substrate of the present invention can be treated to be more biocompatible. For example an apatite material may be applied to the substrate. Furthermore, it is desirable that the substrate encourages growth of local tissue or stimulates cell growth. For substrates implanted into or onto bone, it is desirable that the substrate encourages bone growth. For example any agent that will lead to an increased concentration of osteoblasts within the matrix can be employed. For example recombinant bone morphogenetic protein-2, or other growth factors which induce differentiation of osteoprogenitor cells, can be applied to (coated) on the scaffold e.g. on the surface and may act as an attractant; while fibrin or collagen or other bioglue type materials can act as a medium for the delivery of osteoblast progenitors within the scaffold matrix, which subsequently can act to deposit further bone mass, thus increasing the inter-locking properties of the implant with the host bone.

The invention described herewith involves the development of porous titanium scaffolds for use in orthopaedic implants of the spine, with controllable porosity, pore size, pore shape, pore density and inter-connectivity. The purpose of this invention is two-fold; on the one hand it enables the optimised ingrowth of bone into the porous matrix, which in turn enhances inter-locking of the prosthesis with the host environment, and on the other hand, it enables implant manufacturers to optimise the total amount of metal that needs to be implanted into the body, by ensuring there is only as much metal in the scaffold matrix as is necessary to support the loads to which it is subjected. These two objectives are met, by ensuring a good knowledge of the external loading requirements in the spine, which can be translated into areas of greater metal density and thus lower porosity (in parts of the vertebral body's cross section, where heavier loads are experienced) and areas of lower metal density and thus greater porosity (in parts of the vertebrae with lower external loading). The overall effect of this invention is that it is possible to have spinal implants that do not suffer the same disadvantages as currently available devices, such as spinal cages and screws in terms of stress shielding and consequent non-fusion of the implant with the bone host, whilst also eliminating the need for bone autografts that need to be harvested from a separate surgical site, which is associated with increased risk of infections and peri-operative complications.

The substrate thus has a load bearing profile, which mirrors or replicates the load bearing requirements of the environment in which it is placed.

Additionally alternatively the substrate of the present invention can be used to host therapeutic agents. Examples of such therapeutic agents include those employed to resist or combat infection and those utilised to combat clotting. For example antibiotic agents, optionally in powder form can be employed. This will reduce the risk of early post-implantation infections. Antithrombogenic agents, such as aspirin or warfarin can be employed to reduce the risk of local clots forming immediately after implantation.

It will be appreciated that the elements of the invention described in relation to the substrate, implant and spacer are all interconnected and the disclosure in relation to each applies to the others.

Certain embodiments of the present invention will now be described in detail with reference to experimental procedures and results and the Figures.

### Brief Description of the Drawings

**Figure 1** is a photographic image of a spacer (or scaffold) taken from one side thereof, the spacer constructed from a wax material (as described in Example 1) and being suitable for infiltration with a material for forming a porous substrate;
**Figure 2** is a schematic drawing showing a part-sectional view of a compaction press arrangement suitable for compacting a spacer infiltrated with material for forming a porous substrate - a representation of the spacer of Figure 1 infiltrated with material for forming a porous substrate (the composite) is included within the press;
**Figure 3** is a photographic image (taken from a top side) of a porous stainless steel ("SS") substrate created by the procedure described in Example 1;
**Figure 4** is a scanning electron micrographs image of a porous substrate formed in accordance with Example 1, following removal of the spacer material;
**Figure 5** is a schematic representation illustrating how support structures are employed in certain model building processes;
**Figure 6a -6c** is a schematic representation of a modelling sequence for building a model of a desired spacer structure and the dimensional parameters that can be changed in the computer model, leading to any desirable porosity, pore size, pore shape and interconnectivity;
**Figure 7** is a schematic representation similar to that of Figure 6c, though shown as a side view, and illustrating further dimensions of the model;
**Figure 8** is a photographic image of a spacer made according to the model of Figures 6 and 7;
**Figure 9** is a photographic image of a "hot-wire" cutter utilised to cut the spacer of Figure 8 to a desired shape;
**Figure 10**, is a photographic image of a cylindrical spacer having been cut to the desired cylindrical shape;
**Figure 11** is a photographic image of the cylindrical spacer of Figure 11 having been infiltrated by metal powder and compacted to form a "green" composite material;
**Figure 12** is a photographic image of the metal substrate formed from the composite of Figure 11, the spacer having being removed and the compacted metal sintered;
**Figure 13** is a schematic drawing showing a part-sectional view of a compaction press arrangement suitable for compacting a spacer infiltrated with material for forming a porous substrate and for applying heat while the material is in the press (and employed in Example 3) - a representation of a spacer infiltrated with material for forming a porous substrate (the composite) is included within the press;
**Figure 14** is a photographic image of the metal substrate formed from the composite of Example 3, the spacer having been removed and the compacted metal sintered;
**Figure 15** is a photographic image of a multiple sample compaction rig suitable for compaction of mutiple spacer/substrate material composites;
**Figure 16** is a photographic image of multiple (5) porous Ti substrates made in accordance with Example 4;
**Figure 17** is a photographic image of multiple (5) porous Ti substrates made in accordance with Example 5;
**Figure 18** is a graphic representation of the temperature/pressure profile over the sintering time;
**Figure 19** is a photographic image of machine employed to determine loads borne, and in particular load distribution, in various body parts , for example a functional spine unit (FSU) about the x y and z axes as described below;
**Figure 20A** shows a distribution of pressure across the surface on an intervertebral disc as measured utilising the machine of Figure 19; and
**Figure 20B** shows distribution of pressures for intradisc measurements at two neighbouring discs that are taken a points 1-8 as shown in the respective insets to the Figure;
**Figure 21A** intervertebral samples being taken; and a distribution profile of failure loads across a verterbral endplate; **Figure 21B** shows corresponding results of failure load tests carried out on these samples
**Figure 22** shows a perspective view of a spinal fusion device that can be made in accordance with the present invention with a desired porosity, firstly by itself and secondly after implantation between veterbral bones;
**Figure 23** shows a perspective view of non-fusion intervebral disc replacement implants optionally with a desired porosity; which allow relative movement of the verterbrae between which they are located;
**Figure 24** is a schematic representation a porous spinal implant for insertion between neighbouring veterbrae;
**Figure 25** is a schematic represenation of the spinal implant of Figure 24 showing the desired distribution of pores (within one part of the implant, but the distribution is) continued across and through the substrate;
**Figure 26** is a schematic represenation of the spinal implant of Figure 24 showing an alternative desired distribution of pores (within one part of the implant, but the distribution is) continued across and through the substrate; and
**Figure 27** is a a schematic represenation of a hip replacement implant showing a desired distribution of pores in different selection regions of the implant (within each region of the implant the distribution is continued across and through the substrate).

### Detailed Description of certain Embodiments of the Invention

Figure 1 shows an image of a spacer (or scaffold) taken from one side thereof. The spacer is constructed from a wax material as described in Example 1. It includes a 3D network or array of wax material with a network of pores that have been cut into the matrix. As can be appreciated from Figure 1, the spacer is employed to define the structure of the support substrate created. In particular, the network of material forming the spacer is used to define where the pores will occur in the substrate formed. Similarly, the pores in the spacer accommodate the material for forming the support substrate. In this way, the spacer can be considered to be a negative of the final support substrate. It has pores that correspond to the structural parts of the support substrate and a structural arrangement that corresponds to the pore network in the substrate.

In the experiments described below, the expression Thermojet is utilised to describe a specific model-building machine. While the expression could be considered to relate to a specific printer, the person skilled in the art will appreciate that any machine that can set down the materials for forming a 3D substrate of a desired type can be employed. Most useful within the present invention are machines (often termed printers in this context) which can deposit materials in a 3D arrangement (3DP - "3D printing") to form the structural elements and voids of a spacer of the present invention by an ink-jet type process.

More particularly 3DP is a form of *solid freeform fabrication,* or SFF. SFF refers to a collection of manufacturing processes that build objects layer-by-layer. In SFF, computer software "slices" a 3-D object into a collection of layers by interpreting boundary information. The system uses a technology similar to ink-jet printing, however, the jets in the printhead dispense a molten wax-like material onto a "part bed". A piston that supports the part bed (and the part-in-progress) lowers so that the next wax layer can be spread and added to the previous layer. This layer-by-layer process repeats until the part is obtained.

One issue arising with specific 3DP equipment such as the Thermojet® model is an automated process for taking correctional action to prevent a part being created from falling over or becoming structurally unsound. Generally speaking the system will compensate when it sees the possibility that one or both of these problems may arise.

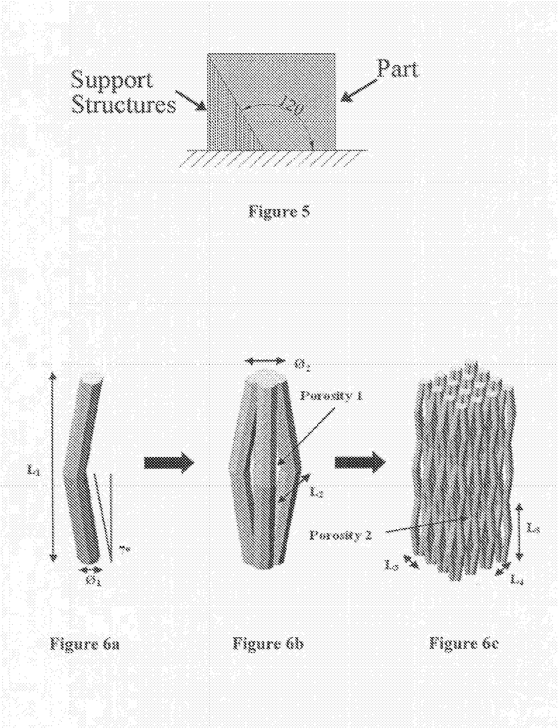

In the Thermojet® system remedial action is taken when an overhang of greater than 7° occurs in a model (c.f. Figure 5) the Thermojet® lays down "support structures" to allow wax to be laid down at this location and also, to prevent the part from falling over. It is for this reason that the models (and thus spacers) in the Examples constructed from struts created at an angle of 7° from the perpendicular as depicted in Figure 6. It is generally desirable that the spacer or structure is constructed from a series of interconnected struts. One desirable arrangement for those interconnected struts is a zigzag (including a herringbone or chevron pattern) arrangement.

It is recognised that one of the critical factors for bone ingrowth into a porous implant 30 is the size of interconnecting pores. Although optimum pore size required for implant fixation remains undefined, the consensus seems to be that the optimal pore size for mineralised bone ingrowth is 100-500µm. For this reason, in the experimental section the diameter of the proposed interconnecting struts (indicated as Ø1 in Figure 6) was set to 500µm.

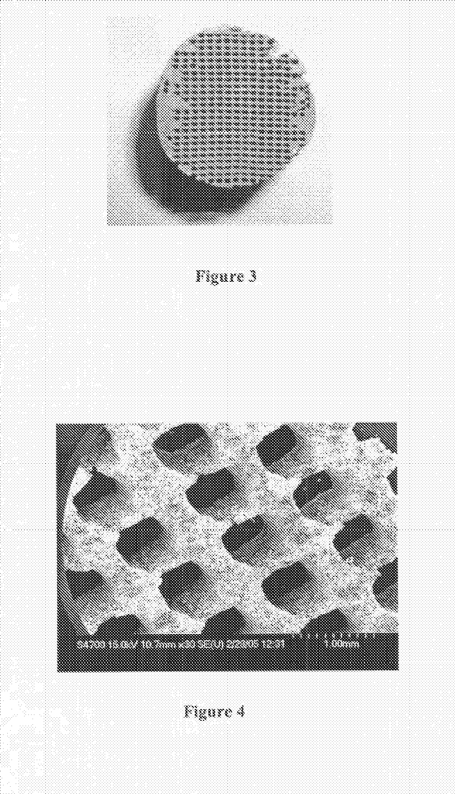

Figure 2 shows split die 6 arrangement with the porous wax and metal powder 4 composite prepared according to Example 1 in place between an upper and lower punch 5 (and inside the walls of the split die 6). A hydraulic press 1 is employed to impart a desired compaction force (indicated by arrow F) via a slug 3 accommodated within the die, to the wax and metal powder composite. Figures 3 and 4 are respectively a photograph of a porous stainless steel substrate; and a scanning electron micrograph image of that porous substrate following removal of the spacer material as set out in Example 1. As can be seen Figure 4, the spacer has been removed without residual material being left behind. Figure 5 illustrates a schematic representation illustrating how support structures are employed in certain model building processes. In particular, in the printing techniques of the present invention, as described below in the Examples, the additional support structures are used to support the material being printed. The pores are created by alternate substrate portions (or struts) which extend (one after another) generally along the same axis but which alternately turn (for example in a zigzag manner) toward and away from that axis.

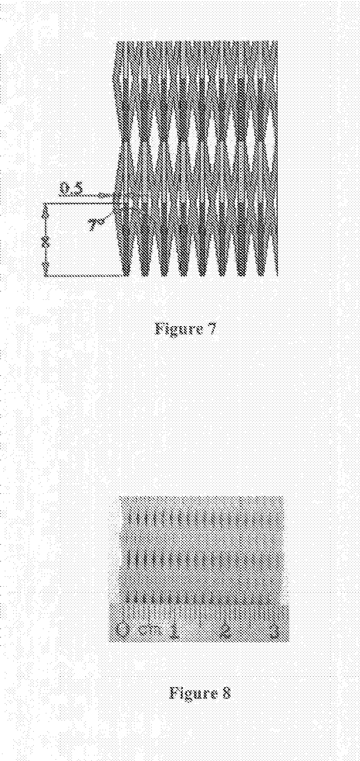

Figure 6a -6c illustrates one schematic representation of a modelling sequence for building a model of a desired spacer structure. It will be apparent to the person skilled in the art that other structures can be employed to give the same effect as that illustrated in Figures 6 (and Figure 7 below). In particular, the following types of structures can also be employed within the present invention: Use of octagonal struts instead of hexagonal, or ideally even cylindrical. Further changes of the structures can include altering the degree of the angle from 7° to higher, for example 10°.

Figure 7 is a schematic representation of the model built-up as shown in Figure 6 and further illustrating additional dimensions that may be employed. Within the types of structures discussed above, the following techniques can be employed: In order to modify the shape, size and interconnectivity of the resulting pores, one can change the parameters of the struts that are used for this purpose. Thereby, the angle can be modified between 7° and 12°. The strut thickness can be varied between 0.25 and 0.5 mm. The height can be varied between 6 and 10 mm. Finally, the cross section of the struts can be changed to octagonal, or cylindrical.

Figures 8 through 10 show how a cylindrical wax model can be made according to the methods described in the present invention. While specific methods are disclosed in the experimental work below, it will be apparent that any three-dimensional forming process which allows the reproduction of a desired (modelled) pore size and distribution can be employed with the present invention. Printing techniques which allow the formation of three-dimensional arrays (suitable for imparting a desired pore distribution to the material forming the substrate) are of particular interest within the scope of the present invention.

Suitable materials for forming the spacer employed in the present invention include those described above.

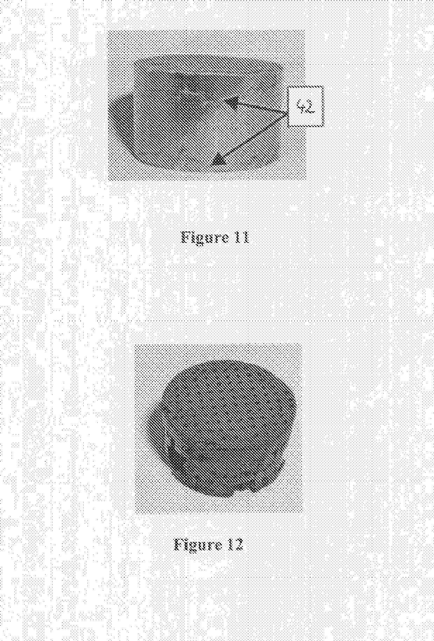

Figure 11 illustrates a "green" composite material which is the spacer material together with the compacted metal powder. While the composite material may suffer from cracks or stress fractures following compaction, but elimination of such undesirable fractures is achieved in later experimental work.

Figure 12 is a photographic image of the metal substrate formed from the composite of Figure 11, the spacer having being removed and the compacted metal sintered in air. The substrate formed has become blackened due to sintering in air. As described below in the experimental section of the present application, formation of black materials, such as oxides, can be eliminated by sintering in a reduced oxygen (air) environment, for example under vacuum or gaseous atmosphere if appropriate.

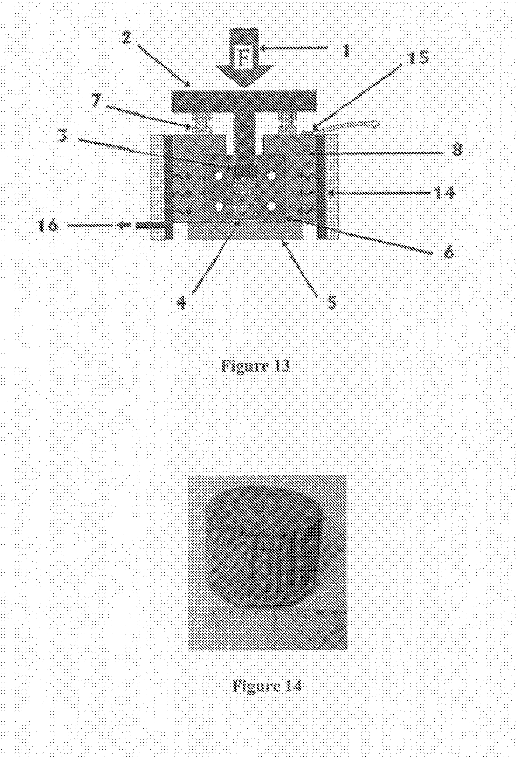

Figure 13 is a schematic drawing showing a part-sectional view of a compaction press arrangement similar to that shown in Figure 2. More particularly, the compaction press arrangement includes a split die 6 arrangement with the porous wax and metal powder 4 composite prepared according to Example 3 in place between an upper 2 and lower punch 5 (and inside the walls of the split die 6). A hydraulic press 1 is again employed to impart a desired compaction force (indicated by arrow F) via a slug 3 accommodated within the die, to the wax and metal powder composite. As indicated in the drawing, the upper punch 2 has been fixed in place run by stopper bolts 7. Accordingly, all of the hydraulic press force imparted to the composite material, is imparted by the lower punch 5. Furthermore, it will be appreciated that a heating arrangement has been applied to the press. In particular a band heater, (together with an appropriate insulation layer) has been placed about the press. The band heater 14 is connected to a suitable power supply 16 and can impart heat (as indicated by the wavy arrows) to the die of the press and indeed the wax/metal composite. A thermocouple 15 is employed to read the temperature of the surrounding die. As described in Example 3, the application of appropriate heat to the composite, can eliminate the formation of cracks such as those shown in Figure 11.

Figure 14 is a photographic image (scale in centimetres - as in Figure 8) of the metal substrate formed from the composite of Example 3, the spacer having being removed and the compacted metal sintered. Again the substrate is suitable for use as a fixation device, for example a bone replacement structure.

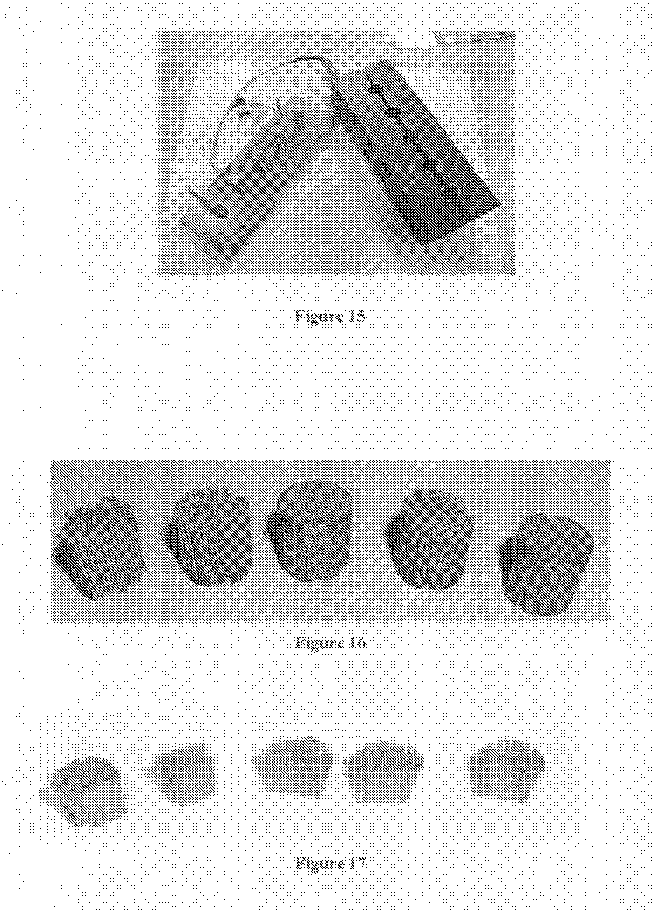

Figure 15 is a photographic image of a multiple sample compaction rig suitable for compaction of mutiple spacer/substrate material composites. Utilising the device of Figure 15, multiple samples can be compacted at any given time. Figure 16 is a photographic image of multiple (5) porous Ti substrates made in accordance with Example 4 while Figure 17 is a photographic image of multiple (5) porous Ti substrates made in accordance with Example 5. Again all are suited for use within all applications of the present invention.

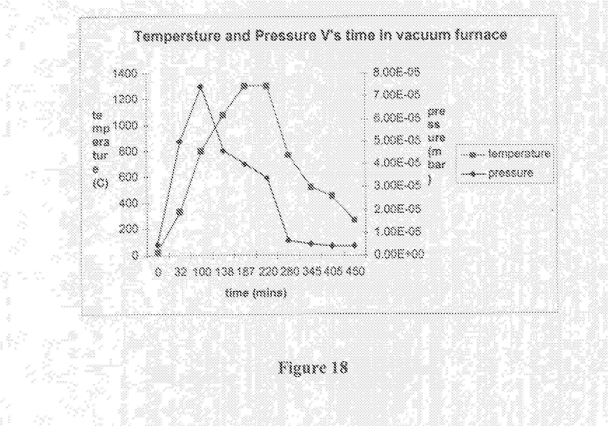

Figure 18 is a graphic representation of a temperature/pressure profile which may be utilised during the time the sintering is taking place. As described in the experimental section below, employing a vacuum during the sintering process can eliminate the formation of impurities such as oxides created during heating.

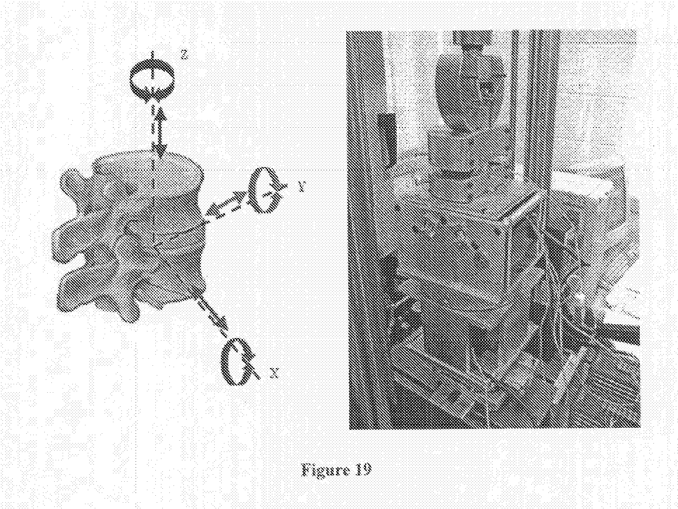

Figure 19 is a photographic image of machine employed to determine loads borne, and in particular load distribution, in various body parts , for example a functional spine unit (FSU) about the x y and z axes. In particular, the machine is employed as described in publication.

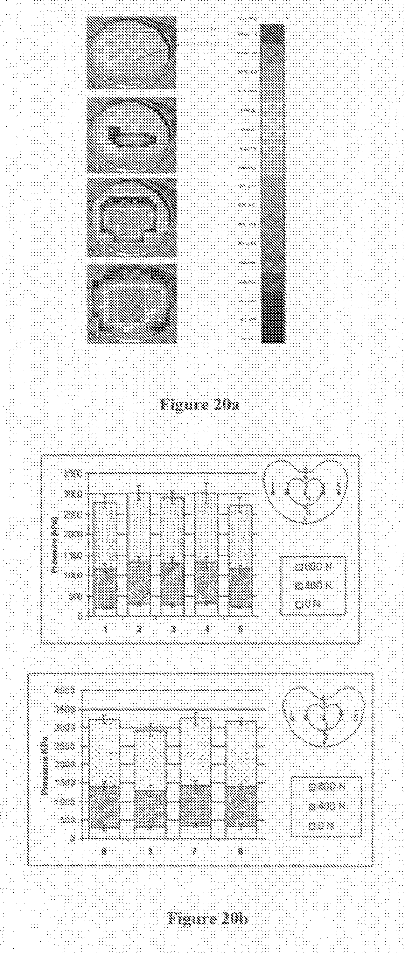

Figure 20A shows a distribution of pressure across the surface on an intervertebral disc as measured utilising the machine of Figure 19; and illustrates a representative load distribution which can be replicated in a support substrate according to the present invention. The combination of the test rig and the present invention allows the loading characteristics required *in vivo* to be modelled and further allows a substrate having a matching load bearing capacity to be manufactured.

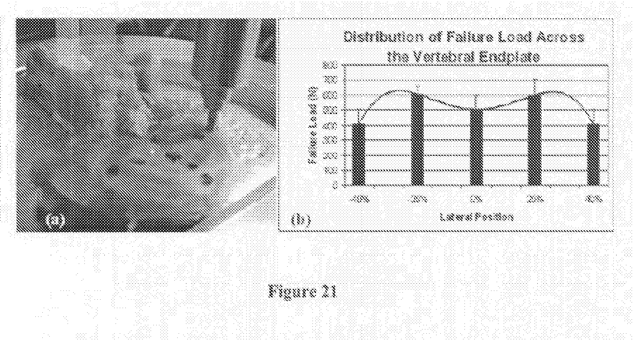

Figure 20B shows some of the intra-discal pressure results that were obtained using the test rig as shown in Figure 19, are presented in Figure 20. The results are an indication of how the distribution of loads at the interface between two neighbouring vertebrae occurs in activities of daily living that were simulated *in vitro.* These activities meant that a series of loading angles were tested as they may occur in vivo when performing different activities, such as walking, bending and rotating the trunk. The conclusion from these tests was that there is a zone of higher loads that occur in the periphery of the inter-vertebral disc, i.e. over the annulus fibrosus, whilst somehow lower loads occur during the same activities in areas that correspond to the nucleus pulposus. This principle is further supported by original data presented in Figure 20A, which was carried out under no loading and two axial loading strengths (400N and 800N). The conclusion was that the optimal adaptation to this anticipated loading situation is to have greater densities of pores and thus lower porosities (~60%) of smaller pores (~200 µm) and therefore thicker metal walls in areas of higher pressures, i.e. those areas corresponding to the annulus fibrosus. In areas of lower pressures, i.e. corresponding to the nucleus pulposus there can be smaller pore densities i.e. higher porosities (~80%) of larger pores (400-500 µm) and thus thinner metal walls. In this way the present technology provides an accurate and reproducible method for fabricating implant 30s with varying porosities that are optimised to the expected loading conditions. This means that only the absolutely necessary amount of biomaterial is implanted, reducing the risk of adverse effects to a minimum. Figure 21 shows how intervertebral samples can be taken and measured in the test bed of the machine of Figure 19

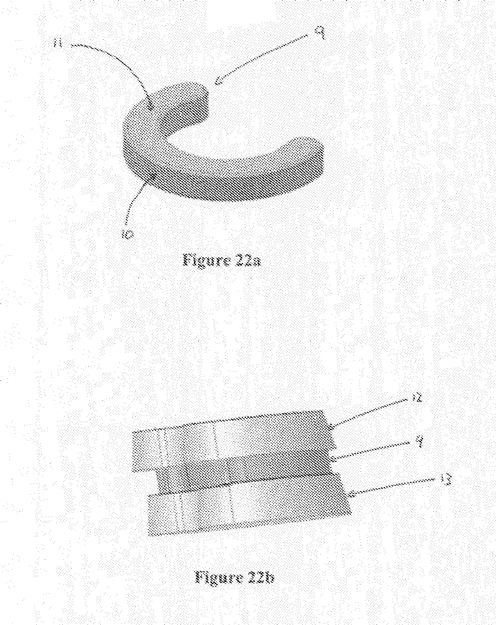

Figure 22a shows a possible variation of a spinal fusion device 9 which is generally a C-shape. The porosity of the implant 30 can be predetermined according to the methods of the present invention. In general though the outer peripheral region 10 of the implant 30 will be under greater loading than the inner region 11. It is therefore desirable that the outer peripheral region is imparted with a porosity which retains sufficient strength in that region while the pore volume fraction in the inner region 11 can be relatively increased thus reducing the amount of structural material (in this case metal) required. Furthermore, in this possible variation, the area corresponding to the overlay of the nucleus pulposus is completely omitted, assuming minimal load bearing involvement, and as an attempt to minimise the total amount of implanted biomaterial. Figure 22b also shows the fusion device 9 in place between two vertebral bones 12,13.

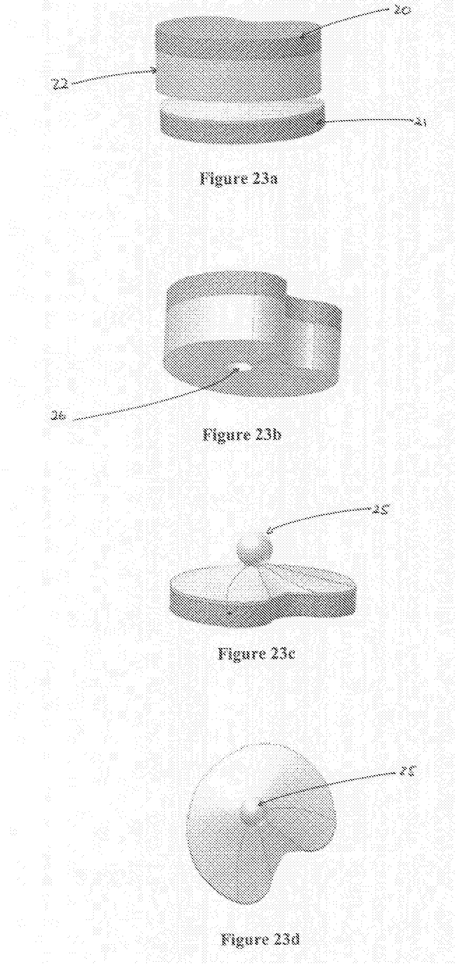

Figure 23a - 23d shows a perspective view of intervebral disc replacement implants optionally with a desired porosity according to the present invention applied thereto. Figure 23a shows three parts to the implants, an upper plate 20, a lower plate 21 and an intermediate spacing plate 22. The upper and lower plates 20;21 are moveable relative to each other. This is an alternative to implants with non-moveable parts such as that described in Figure 22 above and spinal cages which typically fuse the veterbrae. In particular a ball joint 25 fits to a socket 26 to allow the movement. In the embodiment the ball joint 25 (Figure 23c) is on the lower (and upper - Figure 23d) plate 21 while the socket is on the intermediate spacing plate 22 (Figure 23 b) though it will be appreciated that those positions could be reversed. It is desirable, but necessary that the ball and socket interengage to retain the components in their assembled configuration. It will be appreciated that relative movement of the plates 20 and 21 is possible and that such action allows closer to normal body movement as compared to movementless (rigid) implants. Indeed any moveable friction bearing interface can be constructed of or mounted to a porous scaffold and be used as a movement-preserving type implant for example in implants used to preserve a degree of mobility in a FSU. For example the backing plates 20 and 21 can be created by the present invention so that they will fuse with neighbouring vertebral bone.

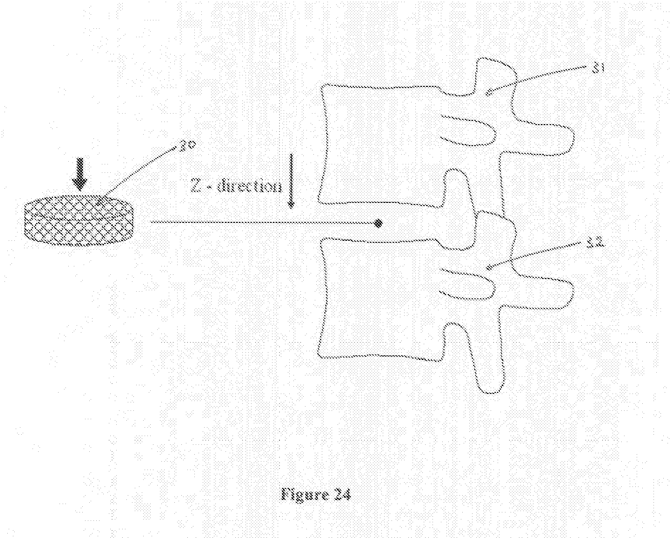

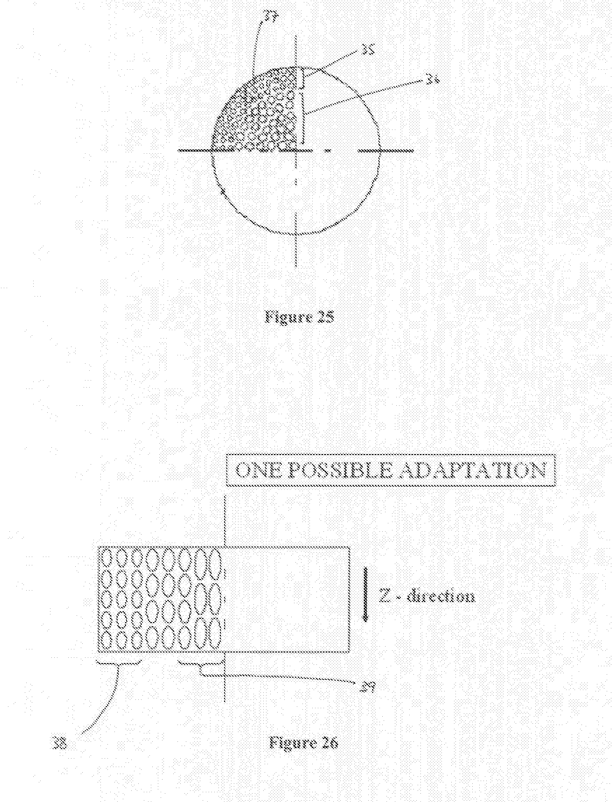

Figure 24 is a schematic representation of a porous spinal implant 30 for insertion between neighbouring veterbrae 31;32. Figure 25 shows the implant 30 of Figure 24 illustrating one desired distribution of pores. For purposes of illustration the pores are shown in one quadrant only, however, the pores are distributed in the same pore network across and through the substrate. In particuar a peripheral band or region 35 comprising a relatively higher number of relatively smaller pores 37 (for example 100 to 150 µm) as compared to a central region 36 comprising a relatively lower number of larger pores. In the embodiment the pore volume fraction is greater in region 36 than region 35.

**Figure 26** is a schematic represenation of the spinal implant 30 of Figure 24 showing an alternative non-uniform distribution of pores (within one part of the implant 30, but the distribution is) continued across and through the substrate. The zone of greater densities and smaller pore sizes 38 has increased strength while the zone of lower density and greater pore sizes 39 has reduced strength. The z direction strength is modified because of the use of ellipsoid pores. Because the longer axes are arranged in the z plane (whcih is the loading direction also) the strength is greater than if the longer axes were in the x,y planes (substantially perpendicular to the axis of loading).

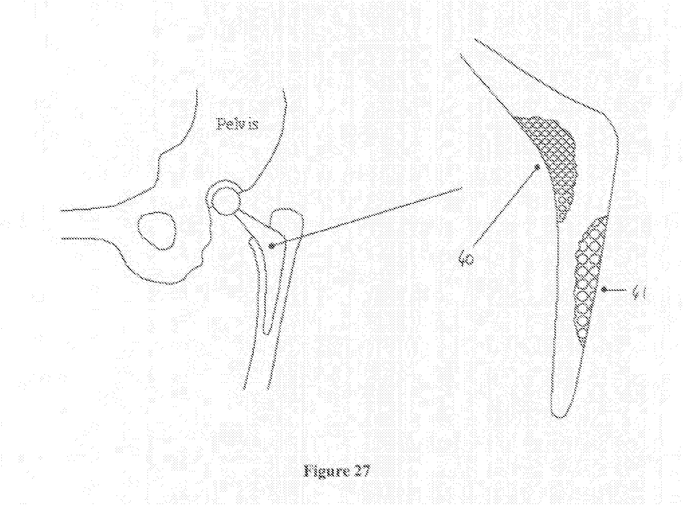

**Figure 27** is a a schematic represenation of a hip replacement implant showing a non-uniform distribution of pores in different selection regions of the implant (within each region of the implant the distribution is continued across and through the substrate). In areas of increased stress concentration 40, (the angular region of the implant and in particular the inside region of that) greater density of smaller pores can be used, while in other areas of lower stress concentration 41, larger pores can be used, whereby the structure has less material.

The present invention allows replication of models created for structural substrates for use, for example as implants within the body. The substrates may be use for any animal but is of most interest in relation to humans.

For example one application of this technology will be as a spinal fusion device, and will form the platform for a total disc replacement implant. This can include the porous scaffold which will allow for integration into the bone environment, and attached to it a centrepiece that will transfer the degree of freedom of the implant. Due to the unique fabrication process developed for creating the porous (Ti) scaffold of the invention, its mechanical properties can be tailored to match those of its intended biological environment. In this way, stress shielding of the surrounding tissue can be avoided.

With this in mind, a series of experiments were performed to determine the appropriate mechanical properties for a porous Ti spinal fusion device, based on the properties of the neighbouring host bone environment. This information was collected as data suitable for transferring to a printer which can then print a 3D structure with a pore network which can incorporate the load bearing requirements.

### Obtaining Data to Produce a Support Substrate

Using a porcine model, experimental protocols were established to analyse the mechanical properties of the functional spine unit (FSU). The FSU consists of two vertebral bodies and their adjoining inter-vertebral disc (IVD). It was hypothesised that the stress distribution through the IVD from one vertebral body to the other is non-uniform and that this results in varying strengths at different loci of the underlying bone. A pressure transducer was used to measure the stress at various locations within the IVD while the functional spine unit was driven into various physiological positions with the help of a custom built spine testing machine (see Figure 19). The machine was provided controlled flexion/extension and medio-lateral flexion movements to the motion segment, while data on intra-discal pressure was simultaneously recorded. The testing machine is comprised of a number of tiers, separated by large deep-groove ball bearings. The cementing pots, into which the FSUs are embedded, are attached to the upper tier with the NP placed at the centre of rotation of a geared platform. The geared platform revolves to introduce the angles of flexion/extension and medio-lateral flexion and can itself be rotated so that varying angles of flexion and medio-lateral flexion can be introduced without disruption of the motion segment. The testing rig was designed for mounting with the Instron 8874 servohydraulic testing machine. This attaches to the upper cementing pot and is capable of applying axial and torsional loads to the motion segment. The companies Imagine, Instron, Zwick and MTS supply such systems.

A LabView® programme was created to control the movements of the machine through its two stepper motors while simultaneously acquiring data from the pressure transducer through a National Instruments® data acquisition device. It was found that while in the normal posture, the stress was evenly distributed between the nucleus pulposus (NP) and the inner two thirds of the annulus fibrosus (AF), but slightly less in the outer third of the AF. However, when medio-lateral or antero-posterior flexion was introduced, the stress increased greatly in the AF, especially in its outer parts, while it remained relatively unchanged in the nucleus. This suggests that bone under the AF needs to be reinforced to sustain the greater stresses present during these movements (Figure 20).

In another experiment, the trabecular bone of the vertebral body below the end plate was sectioned into eight equal 5x5x12mm cubes (Figure 21a). These were compression tested to failure at a rate of 5mm/min using a Zwick® materials testing machine (Figure 21b). Displacement was determined using a video extensometer. Bone underlying the AF was found to have a significantly higher failure strength as compared to bone underlying the nucleus pulposus. The bone was also found to be slightly stiffer on the periphery as compared to the centre. The results are in agreement with the hypothesis that locations within the IVD that are highly stressed lead to underlying bone with higher strength. This is also in accordance with Wolff's law of bone remodelling.

From these findings we concluded that (porous Ti) spinal fusion implants with varying mechanical properties, depending on their loci between the two vertebral bodies, could be employed to avoid stress shielding. If the implants were to cover the entire cross section then its strength could vary over the cross section and it should be stiffer and stronger on the periphery. Using the rapid prototyping method for creating the porous Ti implant it is possible to alter the implant porosity and pore characteristics in such way that the mechanical properties at the various locations are matched.

This invention will form an interesting and more advanced alternative for existing spinal fusion devices, such as rigid spinal cages (Figure 23a), telescopic spinal cages (Figure 23b) and also tapered wedges and screws (Figure 23c). The competitive advantage of this invention over the existing products is that it does not require any bone autografts to be harvested from alternative sites, which normally necessitate additional interventions. Furthermore, this particular invention has the added advantage of being able to minimise the use of implantable metal to a bare minimum, considering that the design is not a solid material, but more importantly, it allows the increase of metal density in areas of higher loading and the reduction in areas that are usually loaded less *in vivo.*

There are currently no alternative products that purport to address the same problem that would include the same advantages. However, products in orthopaedic surgery, such as the Link Prosthesis shown in Figure 24, use porous coatings to ensure that the locking to host bone is achieved by bone ingrowth into the porosities. This anchoring effect is further reinforced by the presence of six 'teeth' on both surfaces. The Link prosthesis of course includes a flexible core, that allows the movement by a few degrees of the neighbouring vertebrae that it is enclosed by. Nonetheless, the porous coated layer in this prosthesis does not follow a particular arrangement, but is of random orientation and density, which means that it is not optimised for total amount of implanted metal, or for strength.

### Example 1

### Porous SS (stainless steel) scaffolds created using Thermojet® support wax as a space holder material.

### Objective:

The objective of this experiment was to determine whether certain wax-based materials such as the Thermojet® wax could be used as a space holder material in the production of a porous metal scaffold.

### Materials and Methods:

Samples of Thermojet® wax support material were acquired from printers of 3D-Systems Inc. (Herts, UK). A cylindrical shape was cut from the support material (see Figure 1) and placed in a custom-made split die 6. 316L stainless steel powder (-325 mesh) was dry poured into the die until the porous wax support material was completely immersed. Using a Dennison® hydraulic press 1, the samples were compacted to 300MPa (see Figure 2 for a schematic representation of the arrangement). After the "green" compact (green is used to refer to a compacted but not yet sintered) was removed from the die it was immersed in a bath of xylene at approx. 60°C for 15 minutes. This process was repeated two additional times using clean xylene. Porous stainless steel with a relatively high porosity was successfully created. Microscopy investigations (SEM investigation - see the image of Figure 4) revealed that the xylene was successful in removing all of the wax space holder material.

### Conclusion

This experiment showed that it is possible to use certain wax materials to form desired space holder or spacer arrangements which can be infiltrated with suitable materials to form a composite suitable for creating a support substrate. The space holder can also be successfully removed (in this case utilising a solvent to dissolve it) without deleteriously affecting the support substrate. Moreover this work showed that it is possible to use a common printing material from a commercially available rapid prototyping machine, and use for the design of porous matrixes, which then can serve as inverse templates for the fabrication of metallic or ceramic matrixes. Xylene was found to completely remove the wax space holder and therefore eliminate the possibility of any contamination of the metal during later sintering of the metal to impart greater structural strength.

### Example 2

### Creation of porous SS scaffold using Thermojet® wax models made to a specific desired porosity.

### Objective:

The objective of this experiment was to create a porous SS scaffold that would have predetermined pore characteristics. This can be achieved by first designing a porous scaffold in a piece of software (in this case AutoCAD®) so that the scaffold (including its pores) is completely pre-modelled as to size, shape and location.

The scaffold model can then be transferred for 3D-printing (3DP), for example to a Thermojet® printer, of the scaffold, utilising in this case a wax material. A similar procedure as described in Example 1 can then be used to create the SS scaffold with the inverse morphology of the porous wax model.

For the reasons discussed above the models constructed for the present Example utilised struts which zig zag at (alternate) angles of 7° from the perpendicular as shown in Figure 6. Indeed Figure 6 shows several of the parameters utilised in the presently described experimental procedure (and which can be applied generally to embodiments of the invention).

Using AutoCAD® 2005 a scaffold was created with the dimensions shown in Figure 7 utilising a stereolithography file or standard template library ("STL"). Figure 6 reflects the sequence and method by which the porous structures were prepared. A unit strut was created using the 7° angle of inclination. The strut veers outwards and turns back on itself (in a general v-shape) to a total height of L₁. The strut cross-section is octagonal to reduce the file size of the STL model.

Using the inner quadrant of a given strut as the base point, a polar array is performed on the strut to create a 4-strut unit structure. This structure has an inner Porosity 1 as indicated in Figure 6b. L₂ is determined by L₁ and also on the position taken for the base point of the polar array. Where the struts meet a larger cross section (⌀₂) is created (see Figure 6b). This unit structure can be arrayed in the X, Y, and Z directions to make a large porous structure. The degree of overlap in each direction as determined by L₃, L₄, and L₅ will create varying thickness of cross section. Another porous region is created where the struts combine.

All these factors can be altered to alter the porosity and pore size of the structure. The dimension L₁ was chosen to be 8mm so that Porosities 1 and 2 would be large enough to allow sufficient SS powder to infiltrate into the porous wax.

An STL file was created using the aforementioned parameters and transferred to the Thermojet® for processing. The resulting wax model is shown in Figure 8 and agreed well with the STL model.

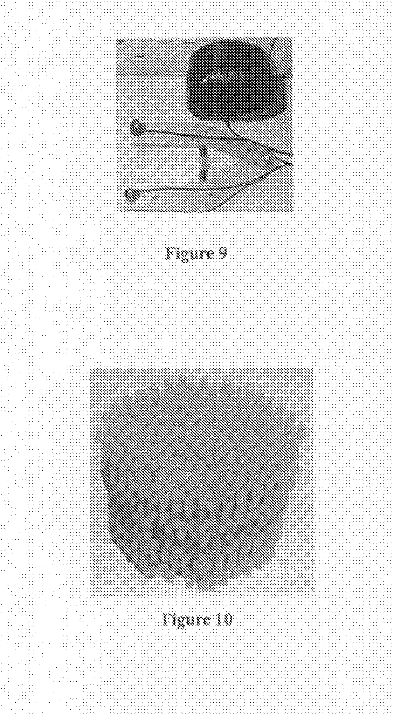

Using a hot wire cutting tool (Figure 9) a cylindrical shape was cut from the wax model and was processed by powder metallurgy method set out in Experiment 1 to yield a porous SS scaffold.

After xylene wax dissolution, the SS scaffold was heat treated to 1200°C in an air environment as described for Example 1 above.

### Results:

Initial filler scaffolds were fabricated using the parameters (such as hexagonal strut cross-section and thickness of 0.5mm) that the Thermojet® system uses when it creates the support structure for 3D printed objects. These parameters were then optimised, according to mechanical and biological requirements, such as pore size distribution as outlined above (150-200µm in high load zones and 400-500µm in zones of lower loading) and pore shape (near spherical or near elliptical).

Figure 11 shows the "green" composite consisting of wax and SS powder. Cracks are clearly visible along the side of the pellet. It was hypothesised that these were due to the difference in mechanical properties between the wax and SS powder. When SS powder is compressed it remains in its new positions when the pressure is removed. On the other hand, the wax being viscoelastic, builds up a reaction force to the compression. When the pressure is removed the wax "springs" back or expands to release this reaction force. This has the net effect of causing cracks (c.f. Figure 11) to develop in the compacted (but not yet sintered) SS powders where they are pushed apart by the expansion of the wax.

Figure 12 shows the SS scaffold after being subjected to a heat treatment of 1 hour at 1200°C. Thick black oxides are present that formed in the furnace atmosphere. Nonetheless, the porous SS was mechanically stable.

### Conclusions:

The cracks in the SS powder that appeared and that were thought to be caused by the expansion of the wax were considered to be undesirable for at least certain applications. A method of eliminating formation of these cracks or of repairing these cracks needed to be determined. Furthermore, the sintering of SS in air was deemed inappropriate due to the presence of thick, and inherently unstable oxide layer on the surface.

### Example 3. Introduction of Heating Element to Compaction Process

### Objective:

The objective of this experiment was to eliminate the presence of cracks in the compacted wax/SS powder pellet following compaction.

### Materials and Methods:

The wax was prepared according to the method described in Example 2, but alterations were made to the compaction rig (c.f. Figure 13), which included a band heater 14 that was placed around the die housing 8 and a thermocouple 15 attached to the housing to monitor its temperature. The rig was positioned in a Dennison® hydraulic press ram and compressed to a pressure of 300 MPa.

At this stage the hydraulic press 1 was changed from load control to position control to prevent the movement of the upper punch 2. Stopper bolts 7 between the upper punch 2 and the die housing 8 ensured the position of the punch was kept fixed. The band heater 14 was then turned on and a thermocouple 15used to monitor the rising temperature of the compaction rig. The temperature was kept at approx. 90°C for 15 minutes. The force exerted by the hydraulic press 1 was then removed and the rig left to cool, after which the wax/SS metal pellet was removed and washed in Xylene for wax removal.

### Conclusions:

The Thermojet® wax has a melting temperature of 70-75°C, which meant it fully melted if the transfer of heat into the wax is sufficient. It is possible that sufficiently softening the spacer (as distinct from fully melting it) may be sufficient. It was shown that the desired process is effective in preventing the formation of cracks in the green composite.

### Example 4. Ti Slurry infiltration of wax to create a porous titanium implant

### Objective:

The objective of this experiment was to replace the metal powder base material to Titanium instead of SS as described in Example 3.

### Introduction:

Ti powder was chosen to replace SS powder due to its better *in vivo* corrosion resistance, and its mechanical properties which are considered closer to those of bone than stainless steel. The inventors hypothesised that this would enable a closer match of mechanical properties of bone when compared to using SS. Commercially pure Ti powder (325 mesh) was purchased from AlfaAesar® and initial experiments were conducted with the aim of infiltrating Ti powder into the Thermojet® wax models. It was discovered that the Ti powder had far different physical properties than the SS powder, which made it clump together. To enable Ti powder to infiltrate the porous wax, a slurry mix of Ti powder and ethylene glycol was used for easing infiltration.

### Materials and Methods:

Porous wax models were created as described in Example 2. A new compaction die and punch assembly was created so that 5 samples could be made per compaction. The porous wax models were placed in the compaction die which was then placed on several sheets of absorbent tissue paper. Titanium powder was mixed with ethylene glycol at a concentration of 1g Ti powder / 1 ml ethylene glycol. The slurry was mixed rigorously using a vortex mixer and then immediately poured into the compaction chambers so that it could infiltrate the porous wax completely. The rig was left for 24 hours so that the ethylene glycol soaked through, attracted largely by the underlying tissue paper. Excess slurry that did not infiltrate the wax was removed from its surface. The process of compaction and heating was the same as that described for SS in Example 3, but this time a rod heating element, as shown in Figure 15, was used to heat the die during compaction and thereby melt the wax. The green wax/Ti powder composites were then removed from the split die 6 and subjected to Xylene wax dissolution as described in Example 1.

### Results:

The experiment was successful in creating five porous Ti samples with controlled architecture as shown in Figure 16. The Ti slurry had successfully infiltrated the wax models and sintered completely.

### Conclusions:

Although the Ti slurry effectively infiltrated the Thermojet® porous wax, further work was carried out to determine what concentration of slurry was most effective in performing this process. This was to ensure that the maximum amount of Ti powder possible was deposited within the wax scaffold and also to ensure that the most appropriate match with the mechanical properties of bone could be achieved, while at the same time strength issues could be adequately addressed.

### 5. Sintering of "green" porous Ti scaffold to form structurally stable samples.

### Objective:

The objective of this experiment was to optimise the sintering of the Ti powder particles and to ensure they would fuse completely and make the scaffolds structurally stable.

### Introduction:

Up to this stage, the Ti scaffolds were being held together through the adhesion forces between powder particles that were created in the compaction process. In this state the scaffolds are extremely brittle and can fall apart to the touch. In order to make the scaffolds structurally stable they need to be subjected to a heat treatment process known as sintering. The sintering process is a solid-state diffusion process where at high temperatures where adjacent powder particles are bonded together with little effect to the overall shape of the structure. A very high vacuum (~10⁻⁵mbar) is required in order to achieve sintering of Ti at high temperatures, ensuring that no oxygen is present in the furnace atmosphere. Any oxygen present could react with the Ti at the high sintering temperatures and form a TiO₂ coating on the particle surfaces, which could inhibit particle sintering and fusion. The utilised system comprised a turbo-drag pumping station from Pfeiffer Vacuum® Ltd, which was attached to a Carbolite® horizontal tube furnace, using specially designed seals. It was confirmed that this system could obtain a vacuum of approximately 10⁻⁵ mbar pressure at a temperature of 1300°C. Alumina boat style crucibles were created for inserting the sample pellets into the tube furnace, protecting them from reacting with the tube walls.

### Materials and Methods:

The utilised furnace was sealed and attached to the turbo-drag pumping station and was left on overnight, whilst the furnace was timed to come on at 9 o'clock the following morning at a heating rate of 5°/min up to 1300°C and held at this temperature for 1 hour. The furnace was then switched off and the samples were left to cool to ambient temperature, but under continuing vacuum conditions.

### Results:

The samples of Ti were successfully sintered and yielded "silver looking" porous scaffolds as shown in Figure 17. They were now structurally stable and seemed strong to the touch.

At the beginning and end of the sintering process the pressure read ~5⁻⁶mbar. However, as the temperature in the tube furnace rose, its pressure also increased to ~7⁻⁵mbar (see Figure 18). This was sufficient to allow complete sintering to occur without the formation of oxides, as was microscopically confirmed.

### Conclusions:

This experiment was the final step in the fabrication process for producing porous Ti with a reproducible micro-structure. It was found that a high vacuum furnace was a good reliable method for sintering Ti. The spacer (wax) structure can be optimised so that the final substrate (e.g. the resulting porous Ti) will have mechanical properties equal to that of bone. This can be done utilising the biomechanical tests that are described above, in particular in relation to Figures 19-21.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A porous substrate for implantation into a human or animal body, constructed from a structural material and having one or more regions which will, in the implanted configuration, be subjected to a relatively lower loading, said region(s) being constructed with lesser mechanical strength.

2. A porous substrate according to Claim 1 wherein said region(s) being constructed with lesser mechanical strength comprise a lesser amount of structural material in said region(s) relative to other regions.

3. A porous substrate for use in a load bearing implant, the substrate comprising:
(i) a load bearing scaffold structure formed of a load bearing material; and
(ii) an open-cell pore network defined by pores in the scaffold structure, the substrate further comprising:
(iii) a first region of higher load capacity; and
(iv) a second region of lower load capacity;
the first region being formed by a load bearing scaffold structure of relatively greater structural strength and the second region being formed by a load bearing scaffold structure of relatively lower structural strength.

4. A porous substrate according to Claim 3 wherein the relatively greater structural strength of said first region is imparted by a lower pore volume in said region relative to said second region.

5. A porous substrate according to Claim 3 wherein the relatively greater structural strength of said first region is imparted by a different pore shape relative to said second region.

6. A porous substrate according to Claim 4 wherein said lower pore volume is formed by having defined in the substrate in said region by at least one of:
pores with a lower relative pore size; a relatively lower number of pores; or a relatively lower interconnectivity of pores.

7. A porous substrate according to any preceding Claim wherein said porous substrate is reticulated.

8. A porous substrate according to any preceding Claim wherein said load bearing material is a metal, for example a metal alloy.

9. A porous substrate according to Claim 8 wherein the metal is titanium or stainless steel.

10. A porous substrate according to any preceding claim further comprising at least a partial coating of a material which is a least one of a cell-ingrowth promoting material.

11. The porous substrate according to claim 10 wherein the cell-ingrowth promoting material is selected from the group comprising such as nucleic acid vectors, growth factors, osteoprogenitor cells, osteoblasts and combinations thereof.

12. The porous substrate according to claim 11 wherein the growth factor is a bone morphogenetic protein.

13. The porous substrate of any one of claims 10 - 12 further comprising a biocompatible material, for example an apatite material, collagen, fibrin and combinations thereof.

14. A porous substrate according to any preceding claim further comprising a bioactive agent, which can act as a chemo-attractant for mesenchymal cells or osteoprogenitor cells *in vivo.*

15. The porous substrate according to claim 12 wherein the chemo-attractant is selected from group comprising fibrin and collagen.

16. An implant comprising the substrate of any preceding claim, for example a fixation device such as an orthopaedic fixation device.

17. An implant constructed of a substrate according to any one of Claims 1 to 16.

18. An implant according to Claim 16 or 17 which is an inter-vertebral disc prostheses.

19. An implant according to Claim 16 or 17 which is a spinal fusion device for example adapted to replace one or more vertebral bodies.

20. An implant according to any one of Claims 16, 18 or 19, which comprises a friction-bearing material sandwiched between two layers of the porous substrate of any one of Claims 1 to 15.

21. An implant according to Claim 19 adapted for the replacement of one or more damaged inter-vertebral discs.

22. An implant according to Claim 16 which is a bone screw such as a dental retention pin, for example for anchoring individual teeth implants.

23. A method of forming a porous substrate for implantation into a human or animal body, from a structural material, comprising the step of (i) identifying one or more regions within a substrate which will, in its implanted configuration, be subjected to a relatively lower loading and (ii) constructing the implant with a relatively lower mechanical strength in said region(s).

24. A method according to Claim 23 wherein said region(s) being constructed with lesser mechanical strength are constructed with a lesser amount of structural material in said region(s) relative to other regions.

25. A method according to Claim 23 or 24 wherein the lesser amount of structural material is achieved by increasing the pore volume fraction in the identified region(s).

26. A substrate constructed by the method of any one of Claims 23 to 25.

27. An implant constructed by the method of any one of Claims 23 to 25

28. A method of forming a porous substrate comprising the steps of :
(i) forming a spacer which is adapted to define an open-cell pore network of the porous substrate;
(ii) infiltrating material to form a load-bearing scaffold structure of the substrate about the spacer; and
(iii) forming the load-bearing scaffold structure with an open cell pore network defined by the spacer.

29. The method according to Claim 28 wherein the spacer is removed prior to forming the load-bearing scaffold.

30. A method according to Claim 28 or 29 wherein forming the load-bearing scaffold structure includes a compaction step.

31. A method according to Claim 30 wherein the spacer is softened or melted by heating during compaction.

32. A method according to any one of Claims 28 to 31 wherein the spacer is removed by extraction utilising a suitable solvent material.

33. A method according to any one of Claims 28 to 32 wherein the spacer is formed by determining at least one region of the substrate that will be required to have relatively greater structural strength and at least one region of the substrate that will that will be required to have relatively lower structural strength and having the spacer impart a relatively lower pore volume fraction in the region of the substrate that will be required to have relatively greater structural strength and a relatively higher pore volume fraction in the region of the substrate that will be required to have relatively lower structural strength.

34. A method according to any one of Claims 28 to 33 wherein the material forming the spacer is printable in a 3D structure.

35. A method according to Claim 34 wherein the material forming the spacer is printed to form the spacer.

36. A method according to Claim 35 wherein the spacer is printed utilising data information which includes data on the regions of required relatively higher and relatively lower structural strength.

37. A method according to any one of Claims 28 to 36 wherein the spacer is constructed of a low melting point solid material such as a wax or synthetic polymer material.

38. A method according to Claim 37 wherein the material has a melting point above 45 °C and below 120°C.

39. A method according to any one of Claims 28 to 38 wherein the spacer material is removable by solvent which is optionally heated.

40. A method according to any one of Claims 28 to 39 wherein the spacer material is a thermoset material.

41. A substrate constructed by the method of any one of Claims 28 to 40.

42. An implant constructed by the method of any one of Claims 28 to 40.

43. A spacer for forming a porous substrate for implantation into a human or animal body the spacer being a three-dimensional array of spacer material for imparting a pore structure to structural material forming the substrate.

44. A spacer according to claim 43 wherein the three-dimensional array of spacer material is configured to impart a higher pore volume fraction to a first region of the substrate and to impart a region of lower pore volume fraction to a second region of the substrate.

45. A porous substrate for implantation into a human or animal body comprising:
a structural material having a pore network defined therein; and having thereon (i) an at least partial coating of an apatite material such as hydroxyl apatite; and (ii) a growth promoter.

46. An implant comprising the porous substrate of Claim 45.

47. A porous substrate according to Claim 45 having the structure of a porous substrate according to any one of Claims 1 to 15.
